# EUROPEAN PATENT SPECIFICATION

(11) **EP 1 899 001 B1**
(45) Date of publication and mention of the grant of the patent: **25.10.2017**
(21) Application number: 06765476.4
(22) Date of filing: 08.06.2006
(51) Int. Cl.: A61N 1/32, A61N 1/40

(54) **APPARATUS FOR TREATING CANCER WITH ELECTRIC FIELDS THAT ARE GUIDED TO DESIRED LOCATIONS WITHIN A BODY**
VORRICHTUNG ZUR BEHANDLUNG VON KREBS MIT ELEKTRISCHEN FELDERN, DIE AUF ERWÜNSCHTE STELLEN IN EINEM KÖRPER GERICHTET SIND
APPAREIL DE TRAITEMENT DU CANCER A L'AIDE DE CHAMPS ELECTRIQUES GUIDES VERS DES EMPLACEMENTS DESIRES DU CORPS

(30) Priority: 08.06.2005 US 688998 P
(43) Date of publication of application: 19.03.2008
(73) Proprietor: Novocure Limited, St. Helier, Jersey (Channel Islands) JE2 4YE (GB)
(72) Inventor: PALTI, Yoram, 34404 Haifa (IL)
(74) Representative: Boden, Keith McMurray
(86) International application number: PCT/IB2006/001500
(87) International publication number: WO 2006/131817

(56) References cited:
- US-A1- 2004 068 297
- US-B1- 6 278 895
- US-B1- 6 334 856

## Description

### BACKGROUND

US-A-6868289 discloses methods and apparatuses for treating tumors using an electric field with particular characteristics. It also discloses various ways to modifying the electric field intensity at desired locations (see, e.g., FIGS. 21-26).

This application discloses additional ways for modifying the field so as to significantly increase or decrease it at desired locations in a patient's body. These modifications can improve the quality and selectivity of treatment of lesions and tumors and improve selective tissue ablation or destruction.

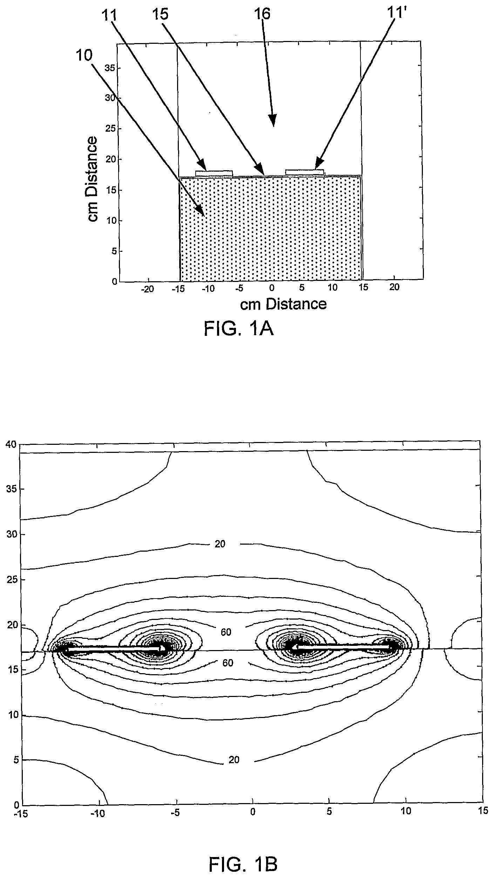

FIG 1A shows an arrangement where two electrodes 11, 11' are placed on the patient's skin 15 above the underlying tissue 10 (e.g., muscle) in an environment of air 16. FIG. 1B depicts the results of a finite element simulation of the electric field generated in the air and in the muscle tissue, when the insulated electrodes 11, 11' are positioned on the skin 15 as shown in FIG. 1A, and a 100 kHz AC signal is applied to the electrodes. Preferably, the insulated electrodes have a conductive core and an insulating layer with a high dielectric constant as described in US-A-6868289, and they are configured to contact the surface of the body with the insulating layer disposed between the conductive core and the surface of the body.

FIG. 1B, (like all the other field intensity maps included herein) shows the field intensity in mV/cm when 1 Volt AC (measured zero-to-peak) is induced between the proximal side of the tissue just beneath the first electrode and the proximal side of the tissue just beneath the second electrode (by applying a sufficiently large voltage between the electrodes' terminals). The numbers along the x and y axes in the main section of FIG. 1B (and in the other field intensity maps included herein) represent distance measured in cm. Each contour line represents a constant step size down from the 1 V peak, and the units are given in mV/cm. Note, however, that because the voltage changes so rapidly at the higher values, the contour lines run together to form what appears to be a solid black region.

It is seen in FIG. 1B that, both in the air above the skin 15 and the tissue below the skin 15, the field intensity is maximal in regions that are close to the edges of the electrodes 11, 11' and that the field intensity is attenuated rapidly with distance. As a result, if a tumor lies relatively deep below the skin 15, it may be difficult to deliver the desired field strength that is needed for effective treatment to that tumor to the target region.

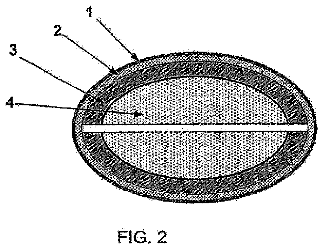

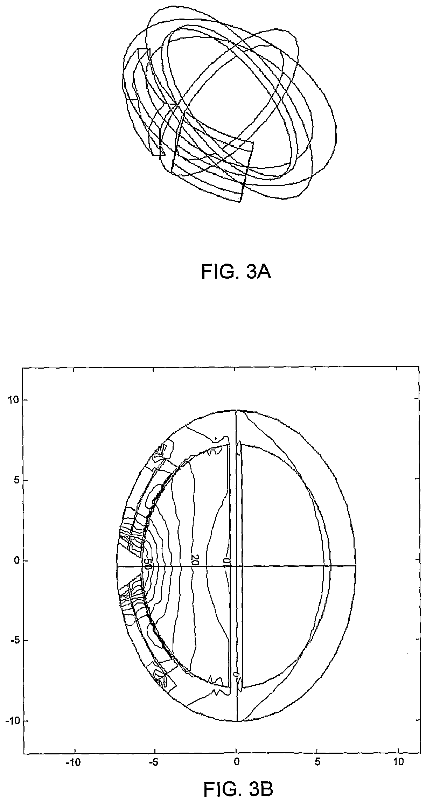

A similar situation exists in the human head. FIG. 2 is a schematic representation of a human head in which all tissue components are given their corresponding electric properties. The head includes skin 1, bone 2, gray matter 3 and white matter 4. FIG. 3A is a schematic representation of the positioning of the electrodes 11, 11' on the skin surface on the same side of the head, and FIG. 3B shows the electric field that is generated under those conditions when a 100 kHz AC field is applied between the electrodes. (The field calculation was done by a finite element simulation based on the schematic representation of the head shown in FIG. 2.) The field intensity is highest in the vicinity of the electrodes in the skin and the superficial areas of the brain and drops rapidly. Notably, the field strength near the middle of the head is very weak (i.e., less than 20 mV/cm).

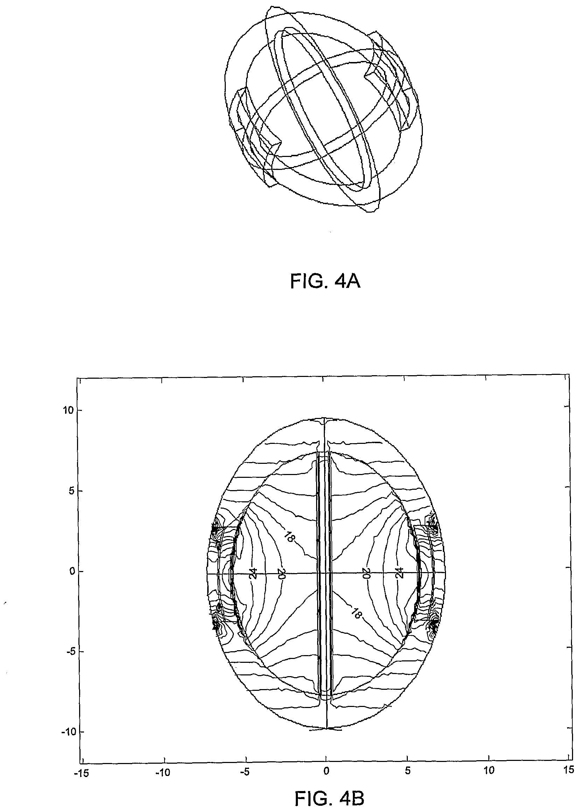

FIG. 4A is a schematic representation of the positioning of the electrodes 11, 11' on opposite sides of a human head, and FIG. 4B shows the electric field that is generated under those conditions when a 100 kHz AC field is applied between the electrodes. Once again, the field calculation was done by a finite element simulation, and once again, the field strength near the middle of the head is very weak (i.e., less than 24 mV/cm). The field intensity is highest in the vicinity of the electrodes in the skin and the superficial areas of the brain and drops rapidly, so that the field intensity is relatively low at the center of the head. Thus, the treatment efficacy of the field for any tumor or lesion at a distance from the surface or electrodes would be correspondingly diminished.

### SUMMARY

A biocompatible field guide is positioned between the surface of the body and the target region beneath the surface. Electrodes are positioned on either side of the field guide, and an AC voltage with an appropriate frequency and amplitude is applied between the electrodes so that the field guide routes the electric field to the target region. In an alternative embodiment, one of the electrodes is positioned directly on top of the field guide.

### BRIEF DESCRIPTION OF THE DRAWINGS

FIG 1A is a schematic representation of two electrodes placed on a patient's skin above a target region.
FIG. 1B shows the electric field that results from the FIG. 1A arrangement.
FIG. 2 is a schematic representation of a human head.
FIG. 3A is a schematic representation two electrodes positioned on the same side of the head.
FIG. 3B shows the electric field that results from the FIG. 3A arrangement.
FIG. 4A is a schematic representation two electrodes positioned on opposite sides of the head.
FIG. 4B shows the electric field that results from the FIG. 4A arrangement.
FIGS. 5A and 5B are section and plan views, respectively, of a first embodiment of the invention using a solid insulated rod.
FIG. 6A shows the electric field that results from the FIG. 5 arrangement.
FIG. 6B is a magnified view of the center of FIG. 6A.
FIG. 7A shows the electric field for a second embodiment using a hollow insulated rod.
FIG. 7B is a magnified view of the center of FIG. 7A.
FIG. 8A shows the electric field for the third embodiment when a conductive gel is added.
FIG. 8B is a magnified view of the center of FIG. 8A.
FIG. 9A shows the electric field for a third embodiment using a hollow conducting rod.
FIG. 9B is a magnified view of the center of FIG. 9A.
FIG. 9C depicts a set of field strength plots for six hollow metal tube field guides.
FIG. 10A shows the electric field that results from using a solid conducting rod.
FIG. 10B is a magnified view of the center of FIG. 10A.
FIGS. 11A and 11B are section and plan views, respectively, of a fourth embodiment of the invention using a solid insulated bead.
FIG. 12A shows the electric field that results from the FIG. 11 arrangement
FIG. 12B is a magnified view of the center of FIG. 12A.
FIG. 13A shows the electric field for a fifth embodiment using a hollow conducting bead.
FIG. 13B is a magnified view of the center of FIG. 13A.
FIG. 14 shows the electric field for a sixth embodiment in which a conductive gel is placed on the surface of the skin between the electrodes.
FIG. 15 shows the electric field for an alternative arrangement in which a rod-shaped field guide is placed directly beneath one of the electrodes.
FIG. 16 shows a curved field guide that guides the field to a target area without passing through a vital organ.

### DESCRIPTION OF THE PREFERRED EMBODIMENTS

The inventor has recognized that the field can be guided to the desired location in the patient's body using appropriate field guides.

In some embodiments of the invention, an insulating member is introduced into the medium or tissue in a position that enables the member to act als a Field Guide (FG) in the given medium. While elongated shapes such as rods, tubes, bars, or threads are preferred, other shapes (e.g., sheets or plates) may also be used. In these embodiments, the electric impedance of the FG, Z_{FG} is significantly higher than that of the medium Z_{FG} (Z_{FG}>> Z_{M}). For example, the FG may be made of a dielectric insulating material such as plastic (e.g. polystyrene, PVC, Teflon), silicone, rubber, etc., while the medium is tissue (e.g., muscle). Insulators with a very high dielectric constant (see the electrode insulations of the '289 patent) may be preferable to those with low dielectric properties. For use in medical application, the FG should preferably be made of a biocompatible material. Optionally, the FG may be made of a biodegradable material, as long as the electrical properties remain as described herein.

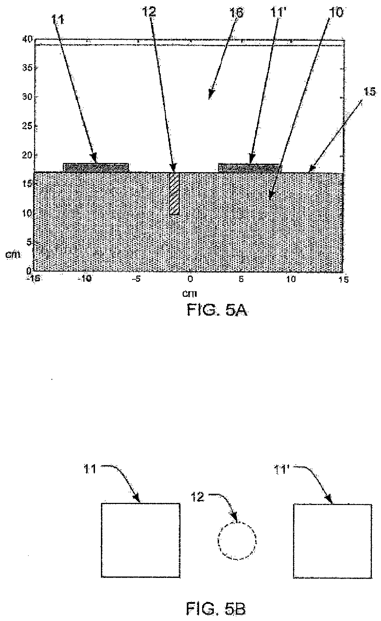

FIGS. 5A and 5B are section and plan views of a first embodiment in which an insulated rod 12 is inserted into tissue 10 between a pair of insulated electrodes 11, 11'. The upper end of the FG rod 12 is positioned just under the skin 15. The preferred diameter for the rod is between about 0.5 mm and about 10 mm, but diameters outside of that range may also be used.

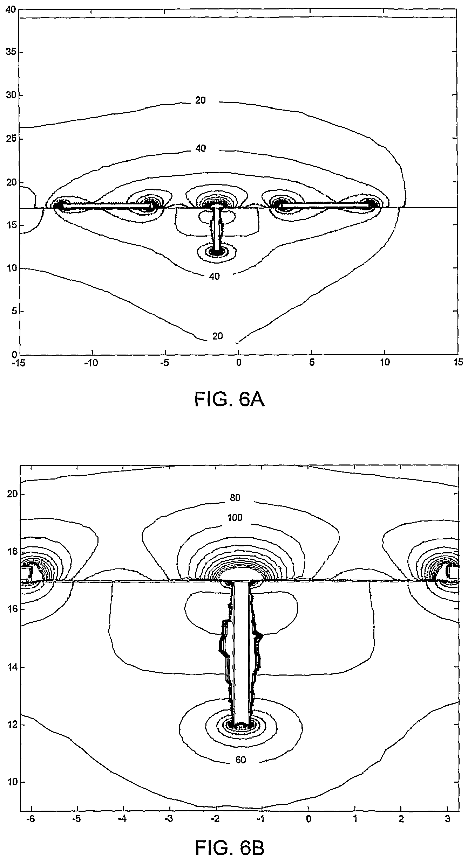

FIG. 6A shows a finite element simulation of the electric field that is generated in the tissue when a 5 cm long, 3 mm diameter, insulated FG rod 12 made of solid plastic with an impedance between 4-6 orders of magnitude higher than the impedance of the tissue and a dielectric constant of about 2-3 is inserted into the tissue 10 between the pair of insulated electrodes 11, 11'. The upper (proximal) ends of the electrodes are located on the skin surface, and a 100 kHz AC voltage is applied between the electrodes. FIG. 6B is a magnified portion of the center of FIG. 6A, to show the field in greater detail. As seen in FIGS. 6A and 6B, the strength of the field is much higher just below the rod 12. Thus, by inserting the FG so that it sits right above the desired target location, the field is directed to the desired location, along with the corresponding beneficial effects of that field (as described in the '289 patent).

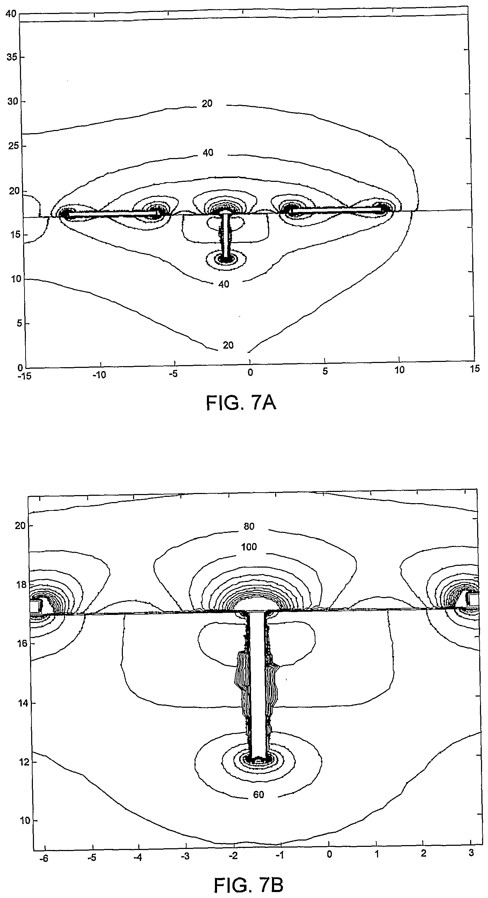

The second embodiment is similar to the first embodiment, except that a hollow insulated rod 12 is used in place of the solid insulated rod 12 of the first embodiment. The rod in this example has an outer diameter of 3 mm and an inner diameter on 2.5 mm, and is also 5 cm long. FIG. 7A shows a finite element simulation of the electric field for this second embodiment, and FIG. 7B shows a magnified view of the center of FIG. 7A. Here again, the strength of the field is much higher just below the rod. We therefore see that a hollow insulating FG can also be used to direct the field to a desired location.

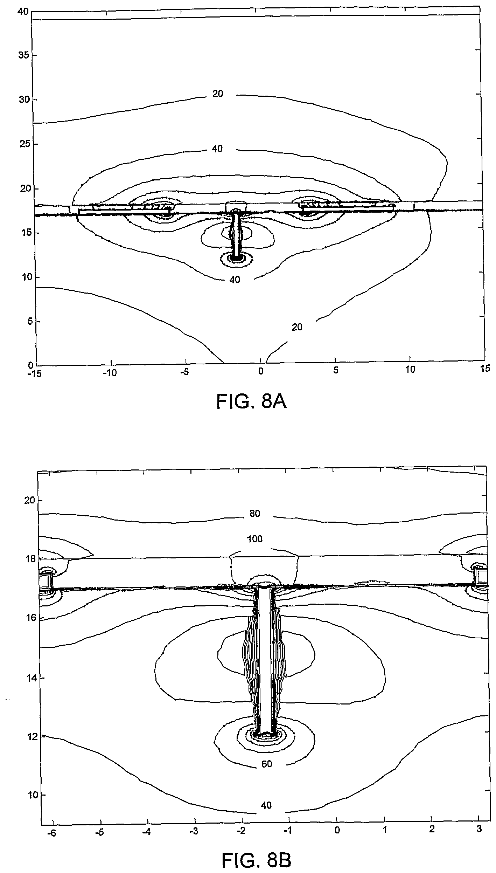

Optionally, conductive gel may be placed on the surface of the skin in the region between the insulated electrodes. FIG. 8A shows a finite element simulation of the electric field for the second embodiment (using the hollow insulated rod 12) when conductive gel is spread on the skin between the electrodes 11, 11', and FIG. 8B shows a magnified view of the center of FIG. 8A. Here again, the strength of the field is much higher just below the rod. In addition, the field is also stronger in the region between the electrodes just below the surface of the skin 15 beneath the gel. Note that the conductive gel described in connection with this embodiment may also be used in the other embodiments described herein, with similar results.

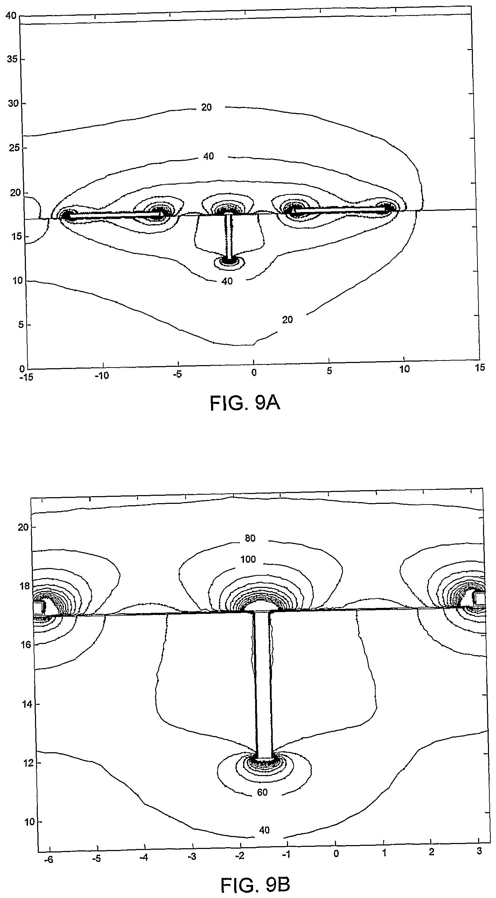

In a third embodiment, a hollow conducting rod is used instead of the hollow insulating rod of the second embodiment. In this third embodiment, the electric impedance of the FG, Z_{FG} is significantly lower than that of the medium Z_{M} (Z_{FG} << Z_{M}). For example, FG may be made of metal such as gold, stainless steel, titanium, etc., while the medium is tissue (e.g., muscle). FIGS. 9A shows a finite element simulation of the electric field for this third embodiment using a hollow conducting rod 12, and FIG. 9B shows a magnified view of the center of FIG. 9B. Here again, the strength of the field is much higher just below the rod 12. We therefore see that a hollow conducting FG can also be used to direct the field to a desired location.

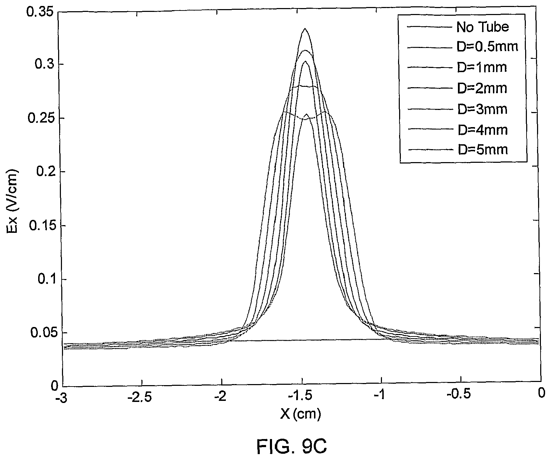

FIG. 9C depicts a set of field strength plots for six hollow metal tube FGs with six different diameters (each having a length of 5 cm) plus a seventh, flat, field strength plot for the case when no FG is used. Each plot depicts how the field strength at the depth of the tube varies as a function of horizontal distance from the center of the tip of the tube. As seen in FIG. 9C, the widest plot corresponds to the tube with the 5 mm inner diameter, and successively narrower plots correspond to tubes with inner diameters of 4, 3, 2, 1, and 0.5 5 mm, respectively. As between the depicted plots, the maximum field strength at the center of the tip of the FG occurs for the 2 mm diameter tube.

In alternative embodiments (not shown), the FG can be of compound construction, such as a hollow metal rod that is coated with insulation or a layer of biocompatible material. In other alternative embodiments, instead of sinking the rod into the tissue to a depth where the top of the rod is just beneath the surface of the patient's skin, a rod that protrudes through the skin may be used with a similar level of effectiveness. In those embodiments, it is advisable to take suitable precautions to reduce the risk of infection.

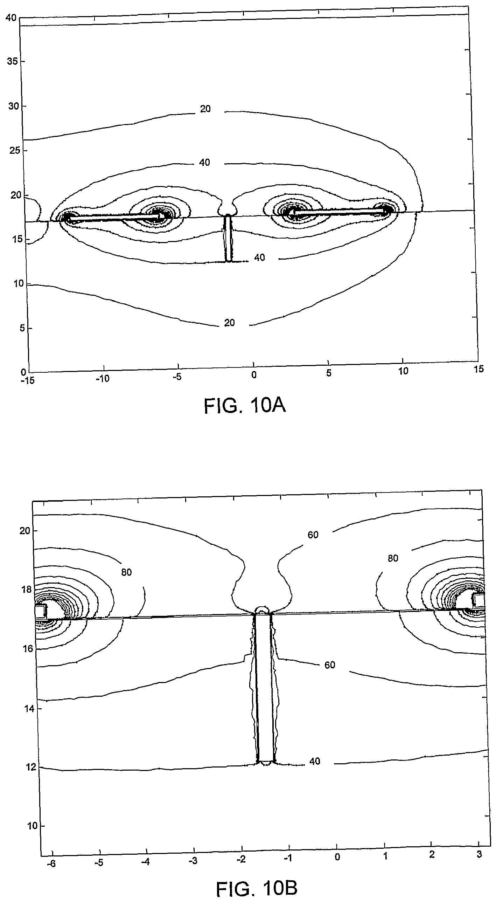

In the above-describe embodiments, the FGs are seen to be effective in carrying the field into deep parts of the tissue. In contrast, if a solid conducting rod 12 were to be used, the field would not be directed to below the bottom of the rod, as shown in the finite element simulation of FIGS. 10A and 10B.

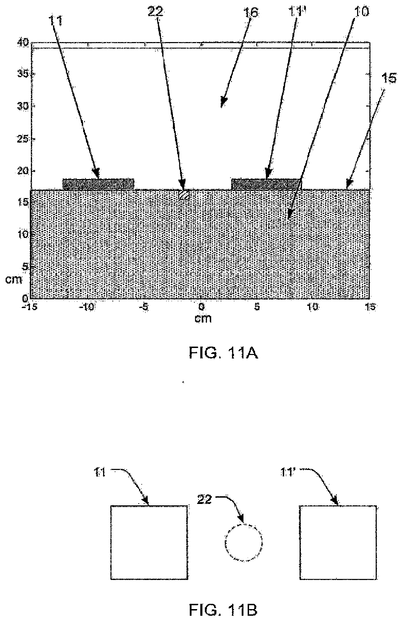

FIGS. 11A and 11B are section and plan views of a fourth embodiment of the invention. In this embodiment, a short insulated solid FG bead 22 is inserted just below the skin 15 between two insulated electrodes 11, 11'. The same materials that are suitable for the insulated FG rod 12 described above in connection with the first embodiment are also suitable for this insulated bead 22. The bead in the illustrated example of this embodiment is cylindrical with a 1 cm length and an outer diameter of 1 cm. Other shapes for the bead (e.g., a cube) may be used as well.

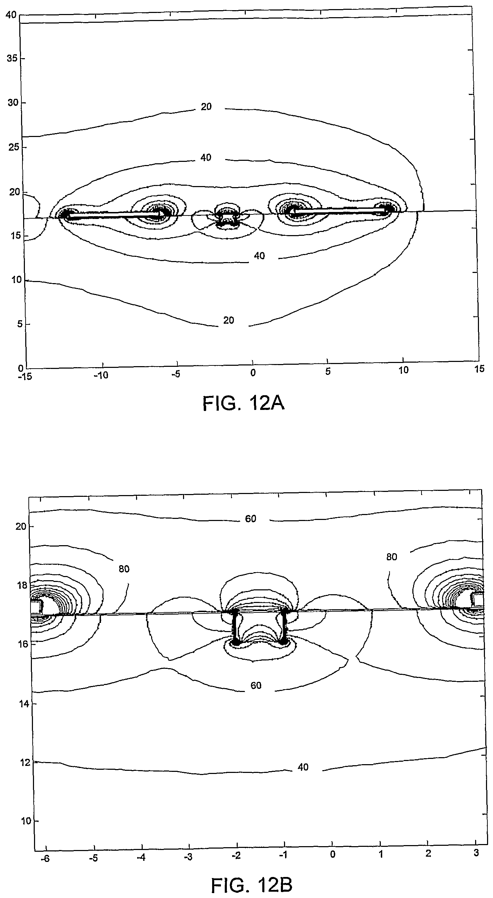

FIG. 12A shows a finite element simulation of the electric field that is generated in the tissue when the insulated bead 22 is inserted beneath the skin into the tissue 10 between the pair of insulated electrodes 11, 11'. The electrodes are located on the skin surface, and a 100 kHz AC voltage is applied between the electrodes. FIG. 12B is a magnified portion of the center of FIG. 12A, to show the field in greater detail. As seen in FIGS. 12A and 12B, the strength of the field is higher beneath the surface of the skin as compared to when there is no FG, as shown in FIGS. 1A and 1B. This embodiment is therefore useful for directing the field into shallow tumors such as malignant melanoma skin lesions or skin metastases from breast cancer, etc.

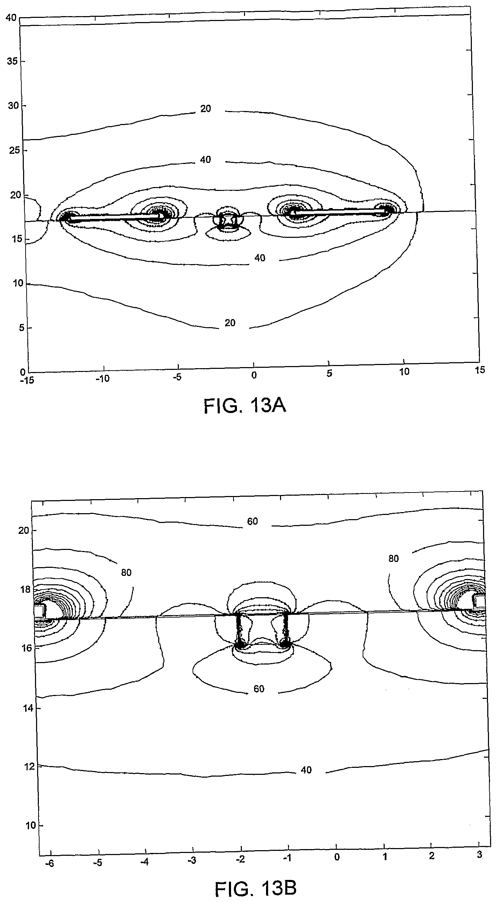

FIG. 13A and 13B show the normal and magnified views of a finite element simulation of the electric field that is generated in the tissue in a fifth embodiment in which the insulated bead 22 of the previous embodiment is replaced with a hollow conductive bead. The field strength in this embodiment is also higher beneath the surface of the skin as compared to when there is no FG, as shown in FIGS. 1A and 1B. This fifth embodiment is therefore also useful for directing the field into shallow tumors.

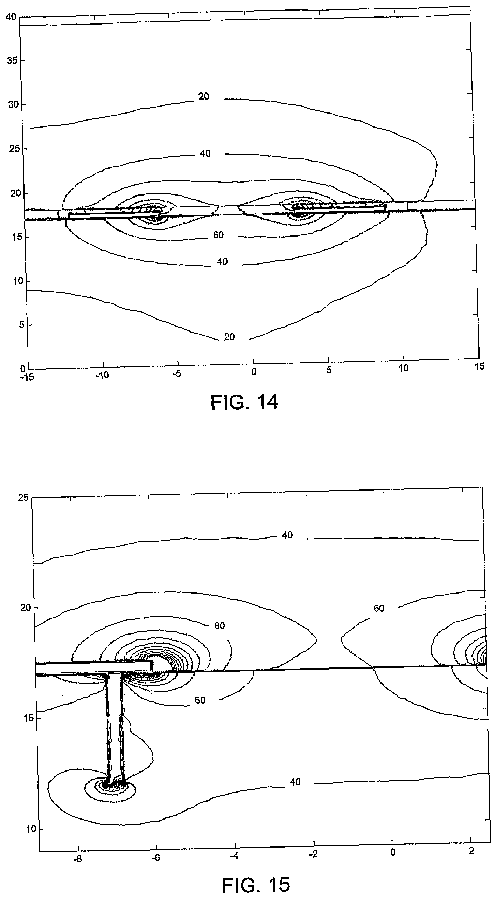

FIG 14 illustrates a sixth embodiment, in which a conductive FG is placed on the skin between the insulated electrodes 11, 11', in parallel with the skin surface. In the illustrated embodiment, the conductive FG is a conductive gel that is spread on the skin in a continuous layer in the region beneath and between the electrodes. Preferably, the gel has high conductivity and is biocompatible for extended periods of time. One suitable gel is AG603 Hydrogel, which is available from AmGel Technologies, 1667 S. Mission Road, Fallbrook, CA 92028-4115, USA. In comparison to the case with no conductive gel (as seen in FIG. 1B), there is a marked intensification of the field in the skin 15 and subcutaneous tissues 10 in the region between the two electrodes 11, 11'.

In a variation of the above-describe embodiments, instead of placing the FG between the electrodes as it is in FIGS. 5-9, an FG rod or bead (which may be either solid insulating, hollow insulating, or hollow conductive, as described above) is placed directly beneath one of the insulated electrodes 11. FIG. 15 shows a finite element simulation of the electric field for this configuration. Once again, the strength of the field is much higher just beneath the FG than it is at a corresponding depth when no FG is used, as shown in FIG. 1B.

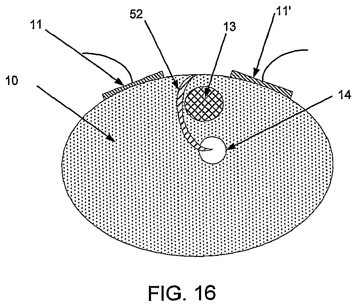

Although straight FGs are depicted in FIGS. 1-10, other shapes may be used in alternative implementations, as appropriate for the anatomy in the vicinity of the tumor. In FIG. 16, for example, a curved FG 52 is used to circumnavigate a vital organ 13 (to avoid piercing the organ 13 with a straight FG) on its way to a target area 14. A thin flexible FG that resembles monofilament fishing line may also be used, in which case it can be threaded into the desired location using a guiding device that is appropriate for the anatomical region.

Superficial FGs may be positioned on the skin surface, under the surface, passing through the skin, or a combination thereof The superficial conducting FG can be a gel sheet, metal sheet, rod tube, etc. The FG can be inserted and maneuvered into position by means of a hypodermic needle, a guided catheter-like device, an incision, etc. Optionally, a combination of active electrodes, superficial FGs, and internal FGs may be used as required to obtain the desired field.

Although the above-described embodiments are explained in the context of increasing the field strength at certain locations in the tissue, a side effect of the FGs is that the field strength is decreased in other areas. This situation can be exploited by using FGs to create areas with lower field intensities so as to avoid effecting, stimulating, or heating sensitive areas within the body or tissue. This provides the ability to protect a sensitive region without depending on the shielding effects of closed or partially closed conductors surrounding an element (such as the conductive net that surrounds a sensitive organ, as described in the '289 patent). Examples of the creation of a reduced-field region in the form of a ring or doughnut can be envisioned by extending the cross sections of FIGS. 6, 7, and 9 out to three dimensions, in which case it becomes clear that a low field area surrounds the FG (as compared to the higher field intensities when there is no FG, as shown in FIG. 1B).

The described use of FGs can increase the efficacy of treating tumors or lesions in many deeply located body locations including, for example, the brain, lung, colon, liver, pancreas, breast, prostate, ovaries, etc. The optimum frequency and field strength will vary depending on the particular problem being treated. For many types of cancers, frequencies between 100 kHz and 300 kHz at field strengths between 1 and 10 V/cm have been shown to be helpful. Examples include B16F1 melanoma, which is susceptible to 120 kHz fields; and F-98 glioma, which is susceptible to fields between 150 and 250 kHz. *See* E. D. Kirson et al., Disruption of Cancer Cell Replication by Alternating Electric Fields, Cancer Research 64, 3288-3295, May 1, 2004. The present invention is set out in the claims that follow:

## Claims

1. An apparatus for inhibiting growth of rapidly dividing cells located in a target region beneath the surface of a body (10), comprising:
an elongate biocompatible field guide having a proximal end and a distal end, and being a dielectric insulator having an impedance that is significantly higher than the impedance of the body (10) or a metal having an impedance that is significantly lower than the impedance of the body (10), wherein the proximal end is configured to be positioned near or above the surface of the body (10) when the distal end is positioned beneath the surface of the body (10) adjacent to the target region and deeper beneath the surface of the body (10) than the proximal end;
a first electrode (11) configured to be positioned on the surface of the body (10) on a first side of the field guide;
a second electrode (11') configured to be positioned on the surface of the body (10) on a second side of the field guide and the same side of the body (10) as the first electrode (11'); and
an AC voltage source configured to generate an AC voltage having a frequency between 100 kHz and 300 kHz between the first electrode (11) and the second electrode (11'), wherein the AC voltage and the impedance of the field guide have values that result in the formation of an electric field in the target region that inhibits the growth of the rapidly dividing cells.

2. The apparatus of claim 1, wherein the field guide is curved.

3. The apparatus of claim 1, wherein the field guide is straight.

4. The apparatus of claim 1, wherein the field guide is a rod, bar or thread.

5. The apparatus of claim 1, wherein the field guide is a flexible monofilament.

6. The apparatus of claim 1, wherein the field guide is a solid insulating rod (12).

7. The apparatus of claim 1, wherein the field guide is a hollow insulating rod (12).

8. The apparatus of claim 1, wherein the field guide is a bead (22).

9. The apparatus of claim 1, wherein the field guide is a solid insulating bead (22).

10. The apparatus of claim 1, wherein the field guide is a hollow insulating bead (22).

11. The apparatus of claim 8, wherein the bead (22) is cylindrical.

12. The apparatus of claim 1, wherein the field guide is a 5cm long, 3 mm diameter insulated rod made of solid plastic and has an impedance between 4 to 6 orders of magnitude higher than the impedance of the body (10) and a dielectric constant of about 2-3.

13. The apparatus of claim 1, wherein the field guide is made of plastic, polystyrene, PVC, Teflon (RTM), silicone or rubber.

14. The apparatus of claim 1, wherein the field guide is a hollow conducting rod (12).

15. The apparatus of claim 1, wherein the field guide is a hollow conducting bead (22).

16. The apparatus of claim 1, wherein the field guide is made of gold, stainless steel or titanium.

17. The apparatus of claim 1, wherein the field guide is made of a biodegradable material.

18. The apparatus of claim 1, wherein the first and second electrodes (11, 11') each have a conductive core and an insulating layer with a high dielectric constant, and the first and second electrodes (11, 11') are adapted to contact the surface of the body (10) with the insulating layer disposed between the conductive core and the surface of the body (10).

19. The apparatus of claim 1, wherein the electric field in the target region has a field strength greater than 1 V/cm.

## Patentansprüche

1. Vorrichtung zur Inhibierung des Wachstums schnell wachsender Zellen, die sich in einem Zielbereich unter der Oberfläche eines Körpers (10) befinden, welche umfasst:
eine längliche biokompatible Feldführung, welche ein proximales Ende und ein distales Ende aufweist, und ein dielektrischer Isolator mit einer Impedanz darstellt, die wesentlich höher ist als die Impedanz des Körpers (10), oder ein Metall mit einer Impedanz, die wesentlich geringer ist als die Impedanz des Körpers (10), worin das proximale Ende ausgestaltet ist, nahe oder über der Oberfläche des Körpers (10) angeordnet zu werden, wenn das distale Ende unter der Oberfläche des Körpers (10) neben der Zielregion und tiefer unter der Oberfläche des Körpers (10) angeordnet wird, als das proximale Ende;
eine erste Elektrode (11), die ausgestaltet ist, auf der Oberfläche des Körpers (10) auf einer ersten Seite der Feldführung angeordnet zu werden;
eine zweite Elektrode (11'), die ausgestaltet ist, auf der Oberfläche des Körpers (10) auf einer zweiten Seite der Feldführung und der gleichen Seite des Körpers (10) angeordnet zu werden, wie die erste Elektrode (11'); und
eine AC-Spannungsquelle, die ausgestaltet ist, eine AC-Spannung mit einer Frequenz von zwischen 100 kHz und 300 kHz zwischen der ersten Elektrode (11) und der zweiten Elektrode (11') zu erzeugen, worin die AC-Spannung und die Impedanz der Feldführung Werte aufweist, die zur Bildung eines elektrischen Feldes in der Zielregion führt, welches das Wachstum der schnell wachsenden Zellen inhibiert.

2. Vorrichtung nach Anspruch 1, worin die Feldführung gekrümmt ist.

3. Vorrichtung nach Anspruch 1, worin die Feldführung gerade ist.

4. Vorrichtung nach Anspruch 1, worin die Feldführung ein Stäbchen ist, ein Stab oder ein Faden.

5. Vorrichtung nach Anspruch 1, worin die Feldführung ein flexibles Monofilament ist.

6. Vorrichtung nach Anspruch 1, worin die Feldführung ein fester, nicht leitender Stab (12) ist.

7. Vorrichtung nach Anspruch 1, worin die Feldführung ein hohler nicht-leitender Stab (12) ist.

8. Vorrichtung nach Anspruch 1, worin die Feldführung ein Kügelchen (22) ist.

9. Vorrichtung nach Anspruch 1, worin die Feldführung ein festes nicht-leitendes Kügelchen (22) ist.

10. Vorrichtung nach Anspruch 1, worin die Feldführung ein hohles nicht-leitendes Kügelchen (22) ist.

11. Vorrichtung nach Anspruch 8, worin das Kügelchen (22) ist zylindrisch ist.

12. Vorrichtung nach Anspruch 1, worin die Feldführung ein 5cm langer, nicht-leitender Stab mit einem Durchmesser von 3 mm aus festem Kunststoff und mit einer Impedanz von zwischen 4 bis 6 Größenordnungen größer als die Impedanz des Körpers (10) und einer Dielektrizitätskonstante von etwa 2-3 ist.

13. Vorrichtung nach Anspruch 1, worin die Feldführung aus Kunststoff, Polystyrol, PVC, Teflon (RTM), Silikon oder Kunststoff ist.

14. Vorrichtung nach Anspruch 1, worin die Feldführung ein hohler leitender Stab ist (12).

15. Vorrichtung nach Anspruch 1, worin die Feldführung ein hohles leitendes Kügelchen (22) ist.

16. Vorrichtung nach Anspruch 1, worin die Feldführung aus Gold, rostfreiem Stahl oder Titan besteht.

17. Vorrichtung nach Anspruch 1, worin die Feldführung aus einem bioabbaubarem Material besteht.

18. Vorrichtung nach Anspruch 1, worin die erste und zweite Elektrode (11, 11') jeweils einen leitenden Kern und eine nichtleitende Schicht mit einer hohen Dielektrizitätskonstante aufweist, und worin die erste und zweite Elektrode (11, 11') angepasst sind mit der Oberfläche des Körpers (10) in Kontakt zu treten wobei die nicht-leitende Schicht zwischen dem leitenden Kern und der Oberfläche des Körpers (10) angeordnet ist.

19. Vorrichtung nach Anspruch 1, worin das elektrische Feld in der Zielregion eine Feldstärke von mehr als 1 V/cm aufweist.

## Revendications

1. Appareil pour l'inhibition de la croissance de cellules à division rapide situées dans une région cible en dessous de la surface d'un corps (10), comprenant :
un guide de champ biocompatible allongé ayant une extrémité proximale et une extrémité distale, et étant un isolateur diélectrique ayant une impédance qui est significativement supérieure à l'impédance du corps (10) ou un métal ayant une impédance qui est significativement inférieure à l'impédance du corps (10), dans lequel l'extrémité proximale est configurée pour être positionnée à proximité ou au-dessus de la surface du corps (10) lorsque l'extrémité distale est positionnée en dessous de la surface du corps (10) adjacente à la région cible et plus profondément en dessous de la surface du corps (10) que l'extrémité proximale ;
une première électrode (11) configurée pour être positionnée sur la surface du corps (10) sur un premier côté du guide de champ ;
une seconde électrode (11') configurée pour être positionnée sur la surface du corps (10) sur un second côté du guide de champ et sur le même côté du corps (10) que la première électrode (11') ; et
une source de tension CA configurée pour générer une tension CA ayant une fréquence comprise entre 100 kHz et 300 kHz entre la première électrode (11) et la seconde électrode (11'), dans lequel la tension CA et l'impédance du guide de champ ont des valeurs qui résultent en la formation d'un champ électrique dans la région cible qui inhibe la croissance des cellules à division rapide.

2. Appareil selon la revendication 1, dans lequel le guide de champ est incurvé.

3. Appareil selon la revendication 1, dans lequel le guide de champ est droit.

4. Appareil selon la revendication 1, dans lequel le guide de champ est une tige, une barre ou un filetage.

5. Appareil selon la revendication 1, dans lequel le guide de champ est un monofilament flexible.

6. Appareil selon la revendication 1, dans lequel le guide de champ est une tige isolante solide (12).

7. Appareil selon la revendication 1, dans lequel le guide de champ est une tige isolante creuse (12).

8. Appareil selon la revendication 1, dans lequel le guide de champ est une bille (22).

9. Appareil selon la revendication 1, dans lequel le guide de champ est une bille isolante solide (22).

10. Appareil selon la revendication 1, dans lequel le guide de champ est une bille isolante creuse (22).

11. Appareil selon la revendication 8, dans lequel la bille (22) est cylindrique.

12. Appareil selon la revendication 1, dans lequel le guide de champ est d'une tige isolée d'une longueur de 5 cm et d'un diamètre de 3 mm en plastique solide et a une impédance comprise entre 4 à 6 ordres de grandeur de plus que l'impédance du corps (10) et une constante diélectrique d'environ 2 à 3.

13. Appareil selon la revendication 1, dans lequel le guide de champ est en plastique, en polystyrène, en PVC, en Teflon®, en silicone ou en caoutchouc.

14. Appareil selon la revendication 1, dans lequel le guide de champ est une tige conductrice creuse (12).

15. Appareil selon la revendication 1, dans lequel le guide de champ est une bille conductrice creuse (22).

16. Appareil selon la revendication 1, dans lequel le guide de champ est en or, en acier inoxydable ou en titane.

17. Appareil selon la revendication 1, dans lequel le guide de champ est en un matériau biodégradable.

18. Appareil selon la revendication 1, dans lequel les première et seconde électrodes (11, 11') ont chacune une âme conductrice et une couche isolante avec une constante diélectrique élevée, et les première et seconde électrodes (11, 11') sont adaptées pour être en contact avec la surface du corps (10) avec la couche isolante disposée entre l'âme conductrice et la surface du corps (10).

19. Appareil selon la revendication 1, dans lequel le champ électrique dans la région cible a une intensité de champ supérieure à 1 V/cm.
